# EUROPEAN PATENT APPLICATION

(11) **EP 0 854 185 A2**
(43) Date of publication of application: **22.07.1998**
(21) Application number: 97203361.7
(22) Date of filing: 22.12.1995
(51) Int. Cl.: C12N 15/11, C07K 14/52, A61K 38/04, G01N 33/53, C07K 14/71

(54) **Assay receptor proteins and ligands**

(30) Priority: 23.12.1994 AU PN0301/94; 23.12.1994 AU PN0300/94
(62) Divisional of application: 95944197.3
(71) Applicant: LUDWIG INSTITUTE FOR CANCER RESEARCH, New York, New York 10105 (US)
(72) Inventor: Stacker, Stephen Alan, Royal Parade, Parkville, VIC 3050 (AU); Wilks, Andrew Frederick, Royal Parade, Parkville, VIC 3050 (AU)
(74) Representative: Fyles, Julie Marie

(57) **Abstract**

This invention relates to growth factor receptors of the receptor protein tyrosine kinase family, preparation of the extracellular domain of the receptor in large amounts, ligands for the receptors, nucleic acids encoding the ligands, and the use of the receptors and ligands in assays.

## Description

This invention relates to growth factor receptors of the receptor protein tyrosine kinase family, preparation of the extracellular domain of the receptor in large amounts, ligands for the receptors, nucleic acids encoding the ligands, and the use of the receptor and ligands in assays.

### Background and Prior Art

Cellular proliferation and differentiation in multicellular organisms requires precise coordination and regulation. Binding of extracellular signalling molecules, such as cytokines and growth factors, to specific cell surface receptors provides one major mechanism by which this is achieved. The receptors span the cell membrane, and are classified as to whether or not they possess an intrinsic protein tyrosine kinase domain in their intracellular region. The tyrosine kinase phosphorylates tyrosine residues in many intracellular proteins, providing an important step in signal transduction. Receptors which have an intrinsic protein tyrosine kinase are known as receptor tyrosine kinases (RTK; growth factor receptor).

With the advent of the Polymerase Chain Reaction (PCR) many novel members of the family have been identified via the highly conserved structural elements within their catalytic domains. Even though these receptors share a common cytoplasmic protein tyrosine kinase (PTK) domain, they have distinctive structural elements in their extracellular domains. These structural elements can be used to classify the RTKs into a given subfamily, and demonstrate the structural diversity that exists within the RTK family (Ullrich and Schlesinger, 1990).

Three RTKs have been studied, named NYK (VEGFR2), tie2 (tek) and RYK, respectively, which were isolated using PCR-based technology (Wilks, 1989). The extracellular domains of these receptors display a diverse array of structural features found in RTKs, and are classified into three distinct sub-families. Neuroepithelial tyrosine kinase (NYK); also called Foetal liver kinase (flk-1) or vascular endothelial growth factor receptor 2 (VEGFR2), is a receptor for vascular endothelial growth factor (VEGF) and possibly for one other factor, and is a class III RTK with seven Ig-like extracellular domains (Oelrichs *et al*, 1993; Terman *et al*, 1992). *tie*2 belongs to a family of RTKs whose extracellular regions consist of an array of two Ig-like domains, three EGF repeats and three fibronectin type III repeats (Runting *et al*, 1993). RYK is an orphan receptor with so-called leucine-rich repeats, but lacks any other identifiable structural motifs (Hovens *et al*, 1992; Paul *et al*, 1992; Stacker *et al*, 1993).

The cellular lining of blood vessels (the endothelium) is an important interface between the blood and the tissues. When a tissue or organ needs to provide itself with a blood supply, a new wave of blood vessel development (angiogenesis) must take place; this requires the division and growth of the endothelium. Of the known proteins capable of recruiting blood vessel development, one particular growth factor, vascular endothelial growth factor (VEGF), is demonstrably one of the most important stimuli to the division of endothelial cells during angiogenesis.

Significant angiogenesis occurs in the adult only in certain situations, such as:
a) the endometrium of the female uterus, shed and re-established during the menstrual cycle,
b) embryo implantation and development,
c) tissue remodelling after wounding,
d) the blood vessels recruited during the establishment of a tumour, a process known as tumour angiogenesis, and
e) diabetic retinopathy, a major complication of diabetes which leads to blindness.

Since tumours are unable to establish themselves without a substantial vasculature, the process of tumour angiogenesis has been a target for the development of anti-cancer drugs. Indeed, the process of tumour angiogenesis is the one thing that all metastatic tumours have in common. However, the turnover rate for normal endothelial cells in the adult is far shorter, and is 12-18 months. Thus an anti-angiogenesis drug would be an important adjunct to any tumour therapy.

It is thought that at least two receptors for VEGF are involved in angiogenesis, but that NYK is the major signalling receptor. Thus, compounds able to bind to the receptor so as to inhibit the activity of VEGF would have a wide range of therapeutic applications. Because angiogenesis is essentially only involved in the situations mentioned above, such inhibitors would be less likely to cause major side effects than conventional medications.

Although there are some agents which are known to inhibit angiogenesis, these known inhibitors act either on a different receptor or via a different pathway. In order to discriminate between inhibitor and non-inhibitor compounds, and to demonstrate specificity of inhibition of the VEGF receptor, *in vitro* assay systems have been developed based on the growth factor VEGF and its receptor VEGFR2 (NYK), which are able to detect inhibitors of the interaction of these two molecules. There are two uses for these systems; first, to establish the sites of interaction between VEGF and its receptor, the better to enable rational design of drugs which will act as either agonists or inhibitors of this process; second, as a natural product screen, wherein the inhibition of the VEGF/VEGFR2 interaction will be evidence for the presence of an inhibitor in a particular natural source.

The methods of the invention utilise the isolated extracellular domain of the NYK protein. A particularly convenient method for production of the isolated extracellular domain is described. However, it will be clearly understood that the invention may use isolated NYK extracellular domain produced by other methods. The method described herein for producing the isolated extracellular domain is convenient and allows production of large quantities of the protein. The receptors are present only in very small quantities normally. Hence our method allows the production of recombinant proteins specifying the extracellular domains of those receptors, to act as affinity reagents for detecting the interactive ligand of interest and also to act as an immunogen for production of antibodies. One of the major considerations when expressing the extracellular domains of receptors is to ensure that the various structural motifs are folded in the correct conformation. Techniques such as simply introducing an in-frame stop codon at the extracellular domain/transmembrane boundary can be used effectively, if monoclonal antibodies which recognise the folded receptor are available for purification of the expressed protein.

Recently, however, the advent of PCR cloning strategies has meant that the complete cDNA sequence is often obtained well before any protein data or monoclonal antibodies are available. This can be circumvented by the use of marker peptides or polypeptides which can be ligated to the protein of interest, and to which affinity reagents are available, such as alkaline phosphatase (Flanagan and Leder, 1990), the Fc region of immunoglobulin (Fanslow *et al*, 1992), glutathione-S-transferase (Smith and Johnson, 1988), and synthetic peptides such as FLAG™ (U.S. Patent No. 4,703,004; Hopp *et al*, 1992) and His-Tag®, T7-Tag™, HSV-Tag™ and S-Tag (Novagen). Some of these markers, especially in applications such as screening for ligands on the biosensor, suffer from the disadvantage that they have their own binding specificity (eg. Fc regions), may interfere with the folding of the domain, or may sterically hinder possible ligand binding sites. In addition, some of these markers have only been developed in prokaryotic expression systems, which may be inappropriate for expression of complex receptors because post-translational modification may be required for activity.

The FLAG™ peptide system was originally described as an N-terminal marker for recombinant bacterial proteins. It is a small, eight-amino acid water soluble peptide, hydrophilic in nature, to which a series of monoclonal antibodies have been made, these are commercially available. The peptide was designed to localise to the outside of fusion proteins and, due to its size, provide minimal interference to the folding of the protein being studied (Hopp *et al*, 1992). To date the majority of studies with the FLAG™ marker have used bacterial expression systems (Hopp *et al*, 1992; Li *et al*, 1992; Su *et al*, 1992; Zhang *et al*, 1991), although one recent study has employed a mammalian expression system (Gerard and Gerard, 1990).

Techniques are described herein which alleviate some of the problems outlined above. A site-directed mutagenesis approach is used to generate restriction sites at the junction of the extracellular and predicted transmembrane domains to facilitate the in-frame ligation of a marker peptide to the ligand binding region of the receptor. In this study the small FLAG™ marker peptide and a CHO cell expression system have been employed for large scale protein production. Soluble fusion proteins were purified from cell supernatants using the commercially available anti-FLAG™ affinity gel, and mild elution with free peptide. This approach has produced pure, functional receptor extracellular domains from a mammalian source. While FLAG™ is specifically referred to herein, it will be clearly understood that other small marker peptides may be employed, using the same site-directed mutagenesis and in-frame ligation method. Monoclonal antibodies to some such peptides are commercially available.

By employing a single site-directed mutagenesis step, we have engineered a restriction enzyme site in the receptor cDNAs to enable the ligation of a pair of oligonucleotide linkers encoding the FLAG™ octapeptide. This avoids the use of PCR on large sections of cDNA (1-3 kb) which often possess regions of high GC content at the 5' end. The FLAG™ octapeptide marker is positioned at the C-terminal end of the extracellular domain, thereby leaving the N-terminal region of the receptor, an area frequently involved in ligand binding, unmodified. This approach has been used to construct FLAG™-fusion proteins with RTKsfrom three distinct subfamilies: VEGFR2, tie2 and RYK. Recombinant fusion proteins expressed in a CHO cell expression system have been purified using anti-FLAG™ antibody affinity chromatography. The proteins were eluted from the affinity column by competition with free FLAG™ peptide, which has minimised their exposure to potentially denaturing agents. This method is generally applicable to expressing and purifying functional extracellular domains of cell surface receptors using a well-characterised marker peptide. This method is particularly useful for studies such as monoclonal antibody production, assays such as immunoassays, biosensor experiments or ligand isolation studies which require functional material.

### Summary of the Invention

In a first aspect the invention provides a method of detecting a ligand able to bind the receptor protein NYK, comprising contacting a receptor protein NYK or a derivative thereof or a functional equivalent thereof with a putative ligand under conditions suitable to allow binding of said putative ligand to said receptor, derivative thereof or equivalent thereof and determining whether binding has occurred.

The putative ligand may be any molecule suspected of being able to bind NYK whether from natural or synthetic origin. In the case of synthetic molecules these may be any synthetic molecule including those of a combinatorial library. In the case of natural molecules, the invention provides a particularly useful method for screening compounds present in rain forest plant extracts, and extracts from marine life such as corals and other marine organisms. The method may also be used for screening compounds derived from plants and animals from other aquatic or terrestrial habitats. Furthermore, the method of the invention may be used initially to screen relatively unpurified compositions or extracts and thus extends to a method of detecting one or more putative ligands in a sample. In such cases the sample suspected of containing one or more ligands is contacted with the receptor protein NYK or derivative or functional equivalent thereof. Said samples may be from the sources described above but also include samples from biological origin such as tissue samples from patients including tumour tissue samples, serum samples and the like.

The term "receptor protein NYK or a derivative thereof or a functional equivalent thereof" refers to naturally occurring NYK proteins including allelic variants thereof, to native NYK which has been modified and to synthetic NYK. The term functional equivalent specifically refers to proteins which retain NYK receptor function. Modification of native NYK includes cleaving the native molecule to obtain the extracellular portion which contains the receptor functions. Synthetically produced NYK is also contemplated and this covers NYK produced by peptide synthesis, for example. Similarly, recombinant NYK is included within the scope of the term and this refers to recombinantly produced NYK including fusion proteins which retain receptor activity. The term also extends to NYK and derivatives or functional equivalents thereof which are expressed on the surface of cells. Preferably such NYK and derivatives or functional equivalents thereof are encoded by a recombinant vector present in said cell.

The term "under conditions suitable to allow binding of said putative ligand to said receptor, derivative thereof or functional equivalent thereof" includes parameters such as a suitable period of time, suitable pH, temperature and other parameters which will be well known to those skilled in the art.

Binding may be determined by any convenient means. For example, either the NYK, the NYK derivative thereof or functional equivalent thereof, or the putative ligand to be tested for binding may be labelled with a detectable marker, such as a radioactive label, a fluorescent label, or a marker detectable by way of an enzyme reaction. Alternatively an *in vitro* bioassay may be used to monitor the function induced by binding. Many suitable detection systems are known in the art. Thus assay systems which are suitable for use in the invention include, but are not limited to, immunoassays such as enzyme linked immunosorbent assay (ELISA); affinity-type assays such as those using coated microtitre plates or slides, or affinity chromatography; fluorescence-activated cell sorting; biosensor assays; and bioassays. In addition to detecting binding, the degree of binding may be measured by one or more of the above techniques.

In a second aspect the invention provides a method of detecting a ligand of the receptor protein NYK in a biological sample said method comprising contacting a receptor protein NYK or a derivative thereof or a functional equivalent thereof with a biological sample suspected of containing said ligand under conditions suitable to allow binding of any of said ligand to said receptor and determining whether binding of said ligand has occurred.

The biological sample may be any biological sample suspected of containing a ligand of NYK, in particular any samples suspected of containing VEGF such as ascites, serum, blood and solid tissues such as tumours and the like. Detection of VEGF is important as this molecule is a marker for tumours and diabetic retinopathy.

The terms "receptor protein NYK, derivative thereof or functional equivalent thereof" and "under conditions suitable to allow binding" have the same meanings as given above.

The method of the invention also extends to the detection of agents which are able to inhibit binding of NYK ligands to NYK.

Accordingly in a third aspect the present invention provides a method of detecting a molecule which is capable of inhibiting interaction of NYK and a ligand comprising contacting a molecule which is suspected of inhibiting said interaction with
(i) NYK or a derivative thereof or functional equivalent thereof and
(ii) a known ligand of NYK
under conditions suitable to allow binding of said NYK or derivative thereof or functional equivalent to said known ligand, and determining whether binding has occurred.

Inhibition of interaction between NYK and its ligand *in vivo* may result in inhibition of angiogenesis or inhibition of vascular permeability.

The terms "receptor protein NYK, derivative thereof or functional equivalent thereof" and "under conditions suitable to allow binding" have the same meanings as given above.

The molecule suspected of being an inhibitor of the NYK ligand interaction may be any molecule which may be an antagonist of this interaction. Such molecules may be derived from natural or synthetic sources such as those described above. The method is particularly useful for screening plant and animal extracts for NYK antagonists. It is important to note that the molecule suspected of being an inhibitor may be a molecule which binds to any region of the NYK protein, not necessarily the receptor site of the protein which has the receptor functions. Any molecule which indirectly inhibits the receptor function may also be detected by the method of the invention. Thus the nature of the NYK protein, equivalent or derivative thereof will to some extent determine the type of inhibitor which is identified by the method of the invention.

Determination of binding may be performed by the methods described above.

Preferably the NYK derivative used in the methods of the invention comprises the extracellular region of NYK, more preferably in the form of a fusion protein.

In a fourth aspect the invention provides a method of detecting a molecule which has an increased ability to stimulate activity of the receptor protein NYK compared to a native ligand of NYK, said method comprising contacting a molecule which is suspected of having said increased ability with
(i) NYK, or a derivative thereof or a functional equivalent thereof
   under conditions sufficient to allow binding of said NYK or derivative or functional equivalent thereof with said molecule and determining whether binding has occurred, and
(ii) comparing said determination with the amount of binding in a control which control comprises contacting (i) and a native ligand under suitable conditions.

The term "molecule with an increased ability to stimulate activity of the receptor protein NYK compared to a native ligand of NYK" refers to any molecule which is capable of bringing about an enhanced effect on the receptor compared to a native ligand. Such enhanced effect will usually be in terms of the ability of the molecule to bring about a better angiogenic response in a bioassay than the native ligand, VEGF. Alternatively the enhanced ability may be in respect of an ability to induce vascular permeability. Such molecules are expected to be useful in pharmaceutical applications where angiogenesis is required such as wound healing and the like. The molecule will generally be a protein. Preferably the molecule is a mutant of wild type VEGF with enhanced angiogenic activity.

The other terms have the same meanings as given above.

In a fifth aspect the invention provides a method of detecting the receptor protein NYK or a variant thereof in a sample comprising contacting a sample suspected of containing NYK or a variant thereof with a known ligand of NYK under conditions suitable to allow binding to occur between said NYK or variant thereof and said known ligand and determining whether binding has occurred.

The term "a variant thereof" refers to naturally occurring alleles and mutants of NYK and to synthetically produced variants of NYK. Such variants retain the functional activity of the native receptor.

The other terms have the same meanings as given above.

The sample may be any sample such as those mentioned above.

The known ligand may be any ligand of NYK and is preferably an antibody, more preferably a monoclonal antibody.

The method of determining binding may be any method such as those described above. In a preferred embodiment the known ligand is coupled to a biosensor chip. This provides a very sensitive assay for the receptor or variants thereof.

In a sixth aspect the invention relates to ligands, inhibitors and agonists identified by the methods of the invention. Preferably said ligands, inhibitors and agonist are in the form of an isolated preparation. This means that they have been purified or separated to at least some degree from the other compounds with which they naturally or usually occur.

In a particularly preferred aspect the invention provides a polypeptide which is a mutant of wild type VEGF and is capable of inhibiting or promoting the activity of the receptor protein NYK by directly binding the receptor or by some other means. The wild type VEGF on which the mutants are modelled may be VEGF of any origin. Preferably the VEGF is of mammalian origin such as human, murine, bovine, ovine, porcine, equine, guinea pig, etc. Different VEGF splice variants may be used as a basis for the mutants. For example murine VEGF exists in different amino acids lengths such as 120, 164, 188 and 206 amino acids (Brier *et al*, 1992). Human VEGF also exists in different amino acid lengths such as 121, 165, 189 and 206 amino acids. In addition homologs of VEGF have been found in a pox virus "orf" (Lyttle *et al*, 1994). Such homologs could also be used as a basis for the mutants. The mutants may be produced by recombinant DNA techniques, direct peptide synthesis or any other convenient technique. Preferably the mutants comprise amino acid deletions, substitutions or insertions in the equivalent of the cysteine-knot motif of murine wild type VEGF. Such amino acid substitutions or insertions may be native amino acids or amino acids from synthetic sources.

Preferably the mutant is an inhibitor of the receptor protein NYK and comprises one or more amino acid substitutions in the region which corresponds to the V3 domain of wild type VEGF. Still more preferably the polypeptides comprise mutations at one or more of the following positions corresponding to mouse VEGF: 73, 111, 117 and 109 to 115. Even more preferably the polypeptides are the mutants K73S, H111G, G117V or VEGF0 described herein or variants thereof having substantially the same biological activity as said mutant. Variants of these mutants may be produced by standard conservative amino acid substitutions. The methods of producing such variants will be known by those skilled in the art.

Alternatively preferably the mutant is an agonist of the receptor protein NYK and comprises a truncated form of wild type VEGF with one or more amino acid substitutions in the region which corresponds to the V3 region of wild type VEGF. More preferably the agonist comprises a truncated protein terminating at or about residue 133 corresponding to wild type mouse VEGF with an amino acid substitution at or around the same position. More preferably the agonist comprises K106*2 as herein described or a variant thereof retaining substantially the same biological activity as the mutant.

In a seventh aspect the invention provides a pharmaceutical composition comprising a ligand, inhibitor or agonist identified by the methods of the invention described above, together with a pharmaceutically acceptable carrier or diluent. The appropriate pharmaceutically acceptable carrier or diluent will be known by those skilled in the art. Similarly methods of producing the pharmaceutical compositions will be known by those skilled in the art. Such compositions may be produced by reference to standard textbooks such as Remington Pharmaceutical Sciences, 17th Edition, Elsevier Publishing Co, Eton, Pennsylvania, USA.

In an eighth aspect the invention relates to an isolated nucleic acid encoding a protein which is a ligand, inhibitor or agonist of NYK, a NYK derivative or a NYK functional equivalent thereof.

The nucleic acid molecule may be DNA or RNA, single or double stranded, linear or covalently closed circular. It may be composed of natural or synthetic nucleotide bases. Those skilled in the art will appreciate that due to redundancy in the genetic code several different codons may be used to encode the same amino acid.

In a particularly preferred aspect the invention provides a nucleic acid molecule which encodes a polypeptide capable of inhibiting or promoting the activity of the receptor protein NYK by directly binding the receptor or by some other means. Preferably the polypeptide encoded is a mutant of a wild type VEGF as discussed above. More preferably said nucleic acid molecule comprises a nucleotide sequence which comprises amino acid deletions, substitutions or insertions in the equivalent of the cysteine-knot motif of wild type VEGF.

Preferably said nucleic acid encodes a mutant which is an inhibitor of the receptor protein NYK and comprises a nucleotide sequence which encodes a polypeptide with one or more amino acid substitutions in the region which corresponds to the V3 domain of wild type VEGF. Still more preferably the nucleic acid molecule encodes polypeptides with mutations at one or more of the following positions corresponding to mouse VEGF: 73, 111, 117 and 109 to 115. Even more preferably the nucleic acid molecule encodes K73S, H111G, G117V or VEGF0 or variants thereof having substantially the same biological activity.

Alternatively preferably the nucleic acid molecule encodes an agonist of the receptor protein NYK which is a truncated form of wild type VEGF with one or more amino acid substitutions in the region which corresponds to the V3 region of wild type VEGF. More preferably the nucleic acid molecule encodes a truncated protein terminating at or around residue 133 corresponding to wild type mouse VEGF with an amino acid substitution at or around the same position. Still more preferably the nucleic acid molecule encodes K106*2 as herein described or a variant thereof retaining substantially the same biological activity.

One particularly rapid and convenient test system for the first, second, third, fourth and fifth aspects of the invention uses an optical biosensor, such as the BIAcore™ (Pharmacia Biosensor AB, Uppsala, Sweden), which enables the use of proteins or peptides immobilised to a sensor chip. The biosensor assay is very simple, reproducible and rapid, while having high specificity. The biosensor assay enables a very high throughput of samples, and is amendable to automation. It is therefore suitable for screening of natural products for their ability to inhibit binding to NYK protein, or for activity as agonists of VEGF. However, it is envisaged that test compounds of a wide variety of structures can be tested using this method.

In a particularly preferred embodiment, the methods of the first, second, third and fourth aspects of the invention comprise the steps of immobilising the extracellular domain of NYK to an optical biosensor chip, and detecting binding by measuring the ability of a ligand compound to bind to the immobilised NYK protein or of a putative inhibitor to inhibit binding of a known ligand, respectively.

In an alternative preferred embodiment, the methods of the first, second, third and fourth aspects of the invention uses a bioassay comprising the step of exposing a cell expressing an NYK extracellular domain as a fusion protein together with a cytokine receptor to a ligand capable of binding to the NYK extracellular domain and determining whether binding has occurred.

According to a ninth aspect, the invention provides a method of production of a recombinant protein having the biological activity of an extracellular domain of a receptor protein tyrosine kinase, comprising the steps of:
a) generating in a nucleic acid encoding the protein a restriction site at the junction of the extracellular and predicted transmembrane domains of the receptor, wherein the restriction site is chosen so that it will be unique to a construct produced in step b,
b) ligating to the nucleic acid produced in a) an oligonucleotide sequence encoding a marker peptide and an in-frame stop codon, optionally with a further unique restriction site at the 3' end of the nucleic acid,
c) expressing the construct produced in b) in a mammalian host cell in culture,
d) isolating said protein by using an affinity reagent specific for the marker peptide, and optionally,
e) subjecting the protein to a further purification step.

Preferably step d) consists of subjecting the culture medium conditioned by host cells to purification although depending on the exact construct and host system there may be other ways of obtaining purification of the protein.

Preferably the marker peptide is FLAG™; however, other small marker peptides are known in the art.

Preferably the host cells are Chinese hamster ovary (CHO) cells, but a variety of other convenient host cells is also known in the art. Preferably the affinity purification step is carried out using affinity chromatography, more preferably with anti-FLAG™ affinity gel and mild elution with free FLAG™ peptide.

Restriction sites at the junction of the domains may be introduced using site-directed mutagenesis or via PCR reaction; the nature of the restriction sites will depend on the specific sequence of the receptor cDNA, but is conveniently a site for a restriction enzyme such as *Bgl*II or *BamH*I. For convenience in selection of recombinant proteins, a unique restriction site, such as *Cla*1, may be inserted at the 3' end of the oligonucleotide encoding the marker protein.

According to a tenth aspect, the invention provides an extracellular domain of a receptor protein tyrosine kinase (extracellular RTK domain), produced using the method of the invention. Preferably the receptor protein tyrosine kinase is selected from the group consisting of neuroepithelial tyrosine kinase (NYK), *tie*2, and RYK. More preferably the receptor protein tyrosine kinase is neuroepithelial tyrosine kinase.

According to a eleventh aspect the invention provides an isolated nucleic acid molecule generated by the method of the first aspect of the invention.

According to a twelfth aspect, the invention provides a monoclonal antibody directed against an extracellular RTK domain. Methods for producing monoclonal antibodies are routine in the art. Such monoclonal antibodies are useful for immunodiagnosis, immunotherapy, immunohistochemistry, and immunoassay.

Compositions comprising an extracellular RTK domain of the invention, or a monoclonal antibody thereto, together with a pharmaceutically acceptable carrier, are also within the scope of the invention.

In the ninth to twelfth aspects of the invention the RTK is preferably selected from the group consisting of NYK, tie2, and RYK, and is most preferably NYK.

### Detailed Description of the Invention

Abbreviations used herein are as follows:
- flk-1: foetal liver kinase-1
- MAb: monoclonal antibody
- MSX: methionine sulphoxide
- NYK: neuroepithelial tyrosine kinase
- PCR: polymerase chain reaction
- RTK: receptor tyrosine kinase
- RYK: related to tyrosine kinases
- tie2: tyrosine kinase with Ig-like domains and EGF repeats
- VEGF: vascular endothelial growth factor
- VEGFR2: vascular endothelial growth factor receptor 2.

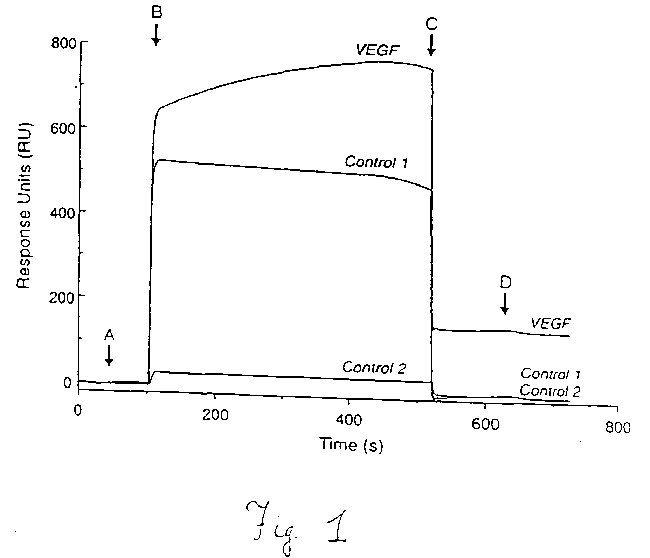

The invention will now be described in detail by way of reference only to the following examples, and to the drawings, in which
Figure 1 shows the results of analysis of VEGF binding to immobilised NYK-EX-FLAG™ using the BIAcore™. Purified NYK-EX-FLAG™ was coupled to a CM5 sensor chip as described herein. The following solutions were then analysed for their binding to NYK-EX-FLAG™; CHO cell medium containing VEGF, CHO cell medium containing tie2-EX-FLAG™ (Control 1) or buffer alone (Control 2). The figure represents the overlay of three sensorgrams representing the relative response units (RU) seen after injection of the above samples over the time of the experiment, measured in seconds. RU relates to the mass change at the surface of the biosensor chip associated with ligand binding. Point A represents the baseline prior to addition of the test samples, B is the point at which the test samples are injected, point C represents the end of the injection phase where the samples are replaced with BIAcore™ buffer and point D, the overall change in baseline due to specific binding of the VEGF.

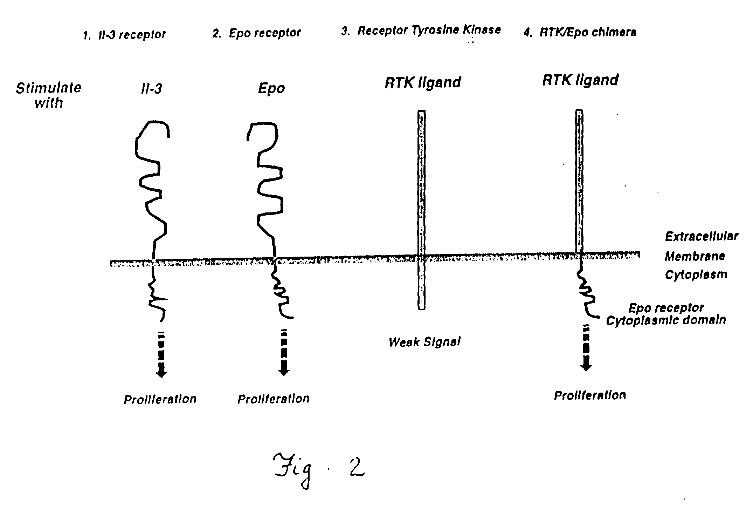

Figure 2 illustrates the mechanism of signal transduction by cytokine receptors, such as those for IL-3 and erythropoietin (Epo), RTK and by an RTK-Epo chimera.

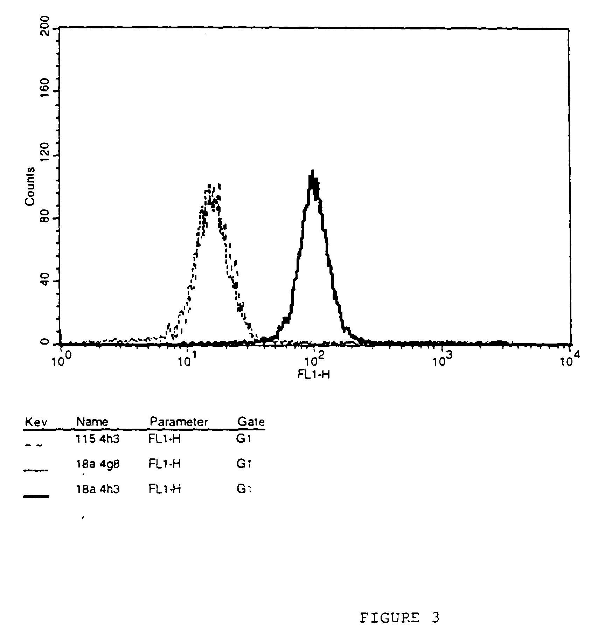

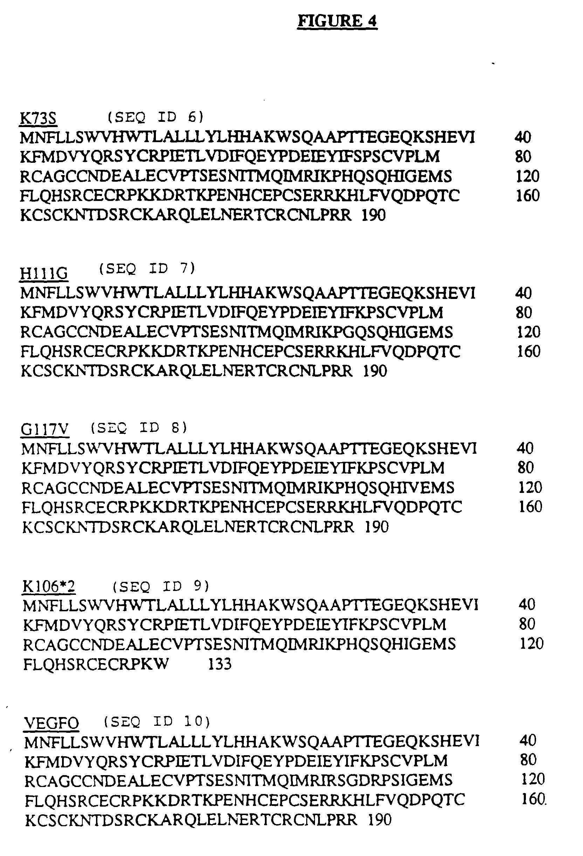

Figure 3 shows flow cytometry analysis of VEGFR2-EpoR expression on the surface of BA/F3 cells using monoclonal antibody 4H3 and control antibody 4g8. The traces are control cell line 115 stained with antibody 4H3 (----) and the BAF/3-NYK-EpoR cell line stained with either 4H3 ( 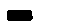 Figure 4 shows the amino acid sequences of modulators of NYK receptor function. The corresponding nucleotide sequences will be well known to those skilled in the art.

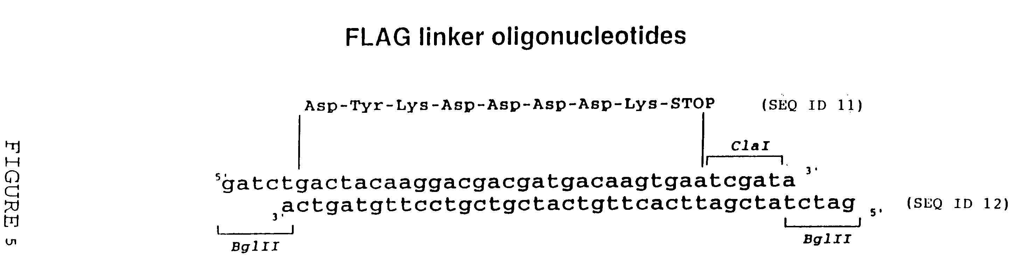

Figure 5 shows the sequence of FLAG™ linker oligonucleotides. The linker sequence encodes the FLAG™ octapeptide, including an in-frame stop codon. *Bgl*II compatible overhangs are present at both ends, to allow ligation into DNA digested with *Bgl*II, *BamH*I, *Bcl*I, *Nde*II or *Xho*II. The internal *Cla*I site allows for identification of recombinants in the ligation reaction.

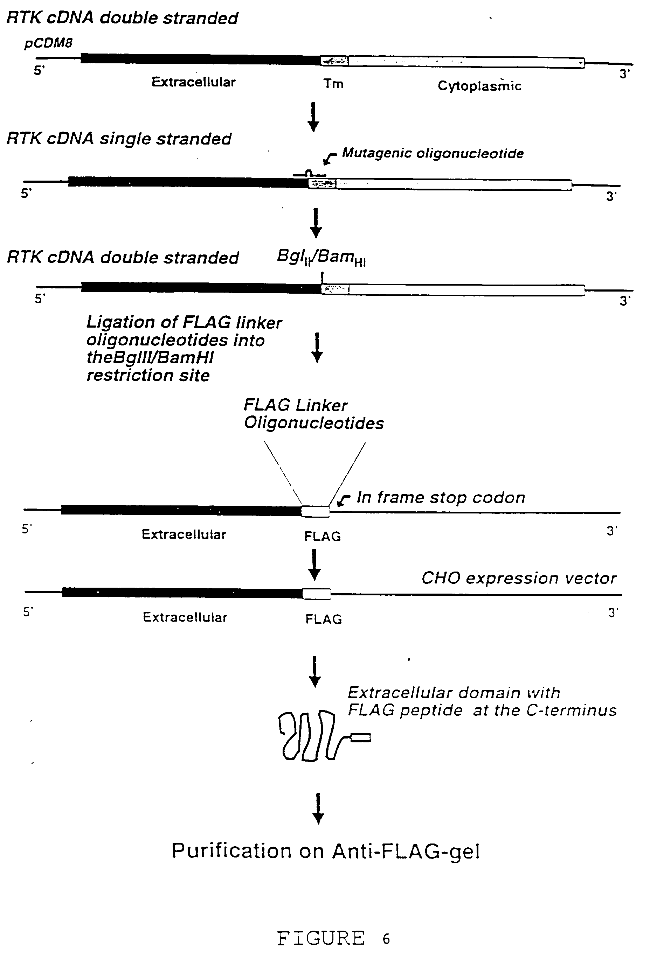

Figure 6 shows a schematic representation of the methods used to make a growth factor receptor extracellular domain-FLAG™ fusion protein. After digestion of the mutant receptor with either *Bgl*II or *BamH*I, the FLAG™ linker oligonucleotides were ligated together and recombinants containing the FLAG™ sequence in frame with the extracellular domain sequence selected. The ability of the FLAG™-fusion protein to be expressed was first assessed by transient expression in COS cells and then the inserts were transferred to a CHO cell expression vector utilising *XbaI*.

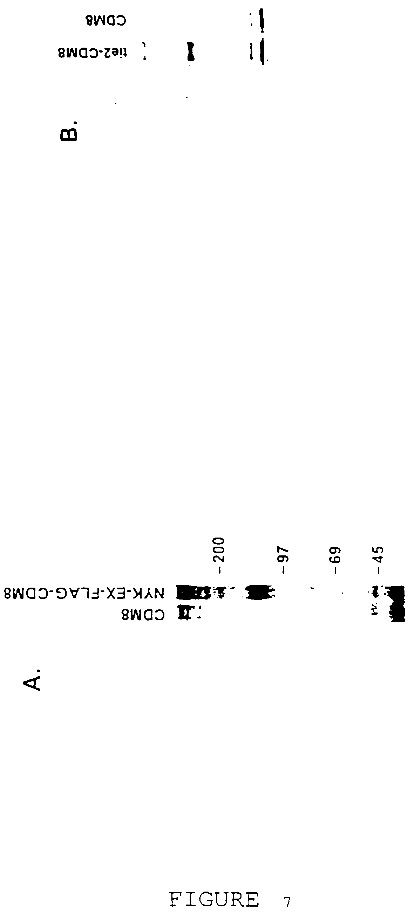

Figure 7 shows the results of transient transfection of constructs into COS cells and detection by immunoprecipitation. Panel (A) shows NYK-EX-FLAG™-CDM8; Panel (B) shows tie2-EX-FLAG™-CDM8; in each case CDM8 alone is used as control. NYK-EX-FLAG™-CDM8 (A), tie2-EX-FLAG™-CDM8 (B) and CDM8 alone (A and B) were transfected into COS cells by the DEAE dextran method. Cells were biosynthetically labelled with ³⁵S-cysteine/methionine for 16 h and the supernatants immunoprecipitated with M2-gel. Washed beads were eluted with SDS-PAGE sample buffer and analysed by SDS-PAGE. The gels were dried and labelled proteins detected by exposing to a storage phosphor screen and analysing using a 400 series Phosphorimager and Imagequant v3.0 software (Molecular Dynamics, Sunnyvale, CA).

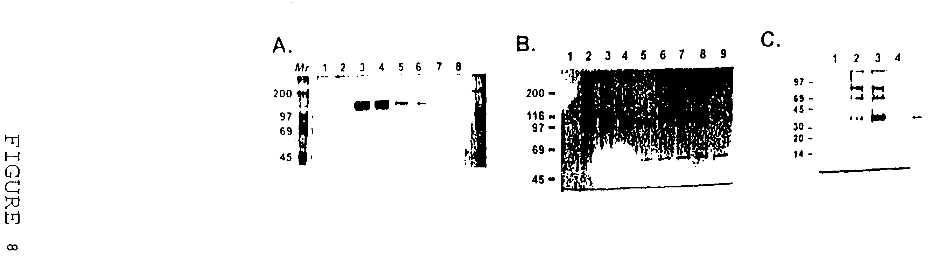

Figure 8 shows the results of analysis of affinity-purified fusion proteins by SDS-PAGE and silver staining. Expended tissue culture supernatant from CHO cell lines expressing (A) NYK-EX-FLAG™ (lanes 1-5 elution with 25 µg/ml FLAG™ peptide, lanes 6-8 elution with glycine-HCl, pH 3.0), (B) tie2-EX-FLAG™ (lanes 1-3 elution with 25 µg/ml FLAG™ peptide, lanes 4 and 5 with 50 µg/ml peptide and lanes 6-9 glycine-HCl pH 3.0 and (C) RYK-EX-FLAG™ (eluted with 25 µg/ml free FLAG™ peptide, lanes 1-4, lane 1=void volume) were passed over independent M2-gel affinity columns and washed as described herein. Elution was performed with free FLAG™ peptide (25-50 µg/ml), or 0.1 M glycine pH 3.0 containing 0.02% Tween 20. The proteins eluted were analysed by SDS-PAGE under reducing conditions and detected by silver staining. Molecular weight markers are indicated.

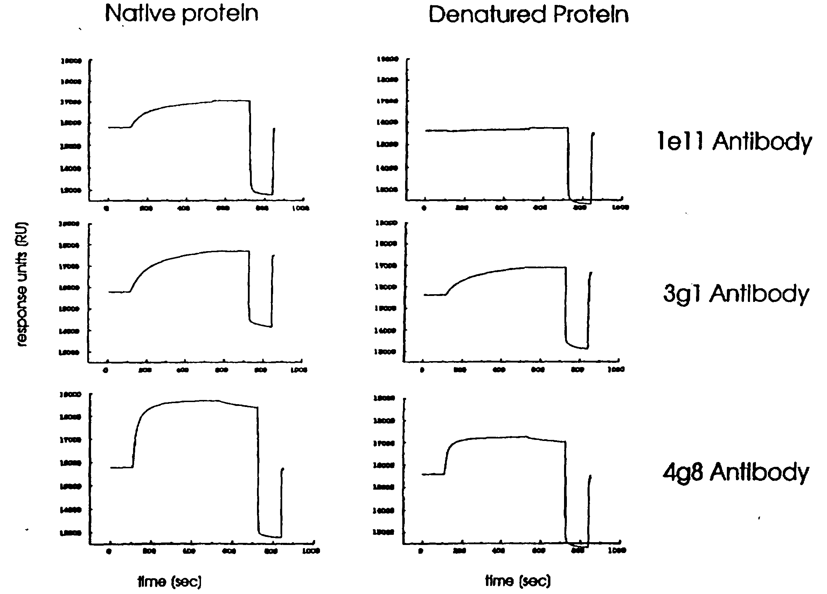

Figure 9 shows the results of analysis of binding of monoclonal antibodies to tie2-EX-FLAG™ to native and denatured tie2-EX-FLAG™, using the BIAcore™.

The following description utilises the extracellular domain of the NYK protein, produced in CHO cells as a fusion protein with the marker peptide FLAG™ (Hopp *et al*, 1992) linked at the C-terminus of the extracellular domain, and purified by affinity chromatography. Production of this fusion protein, designated NYK-EX-FLAG™, is described in Examples 7 to 9. However, it is to be clearly understood that the invention is not limited to NYK-EX-FLAG™, and that NYK, and particularly the extracellular domain thereof, produced by other methods is within the scope of the invention.

### Example 1 Binding Experiments

The NYK-EX-FLAG™ fusion protein was tested for its ability to bind a known ligand, vascular endothelial growth factor (VEGF) (Millauer *et al*, 1993).

Binding studies wore performed on the optical biosensor (BIAcore™, Pharmacia Biosensor AB, Uppsala, Sweden) using proteins immobilised to a CM5 sensor chip (Pharmacia). Immobilisation of NYK-EX-FLAG™ was performed using standard NHS/EDC chemistry as previously described (Nice *et al*, 1994). Approximately 15 ng/mm² (15,000 RU) of NYK-EX-FLAG™ were coupled to the biosensor chip as determined by the BIAcore™ analysis. The integrity of immobilised protein was examined by comparing the BIAcore™ response to medium conditioned with VEGF-pCDM8 transfected CHO cells , tie2-EX-FLAG™ transfected CHO cells and buffer alone. Between each cycle, the derivatised sensor surface was regenerated with 50 mM diethylamide, pH 12.0, 0.1% Triton X-100. In control experiments it was established that this treatment had no effect on the binding capacity of the immobilised receptor for VEGF.

The sensorgrams depicted in Figure 1 show that a sensor chip derivatised with NYK-EX-FLAG™ gave rise to a relative response of 146 RU upon injection of VEGF containing CHO cell supernatants; compare A to D. By comparison, signals from control CHO cell supernatant from cells expressing another RTK protein, tie2-EX-FLAG™ (Control 1) or buffer alone (Control 2) were below 20 RU. The rise in response units seen in the BIAcore™ sensorgram of VEGF binding to NYK-EX-FLAG™ (t=100-520s, B-C) is also consistent with the binding of a specific ligand. This contrasts with the sensorgrams seen for Controls 1 and 2, which show no increase over this period. A panel of monoclonal antibodies which recognise native NYK and tie2 (described in our International Patent Application No. PCT/US95/01743, filed 9 February 1995) also yielded specific responses to NYK-EX-FLAG™ and tie2-EX-FLAG™ derivatised biosensor chips respectively.

Table 1 shows the results of binding of three different monoclonal antibodies, respectively designated 3B6, 3C8 and 4H3 (described in International application PCT/US95/01727 filed 9 February 1995), to NYK-EX-FLAG™ immobilised on the sensor chip. Conditioned media containing the monoclonal antibody at a concentration of 10-20 µg/ml were applied to the sensor chip, and the relative response compared to that shown using buffer alone. The sensor chip was regenerated with a high pH wash between applications.

**Table 1**

| Binding of Anti-NYK Monoclonal Antibodies to NYK-EX-FLAG™ Immobilised on a Sensor Chip | |
|---|---|
| **Monoclonal antibody** | **Relative Response (RU)** |
| 3B6 | 1194 |
| 3C8 | 875 |
| 4H3 | 1163 |
| Buffer | 139 |

Three different monoclonal antibodies directed to tie2-EX-FLAG™, designated respectively 1e11,3g1, and 4g8 were tested for their ability to bind to tie2-EX-FLAG™ immobilised on the sensor chip.

In order to study the effect of denaturation of the tie2-EX-FLAG™ protein on antibody binding, tie2-EX-FLAG™ was immobilised on the BIAcore™ chip. The results are shown in Figure 9. The response to the three purified antibodies, each at the same concentration, on tie2-EX-FLAG™ in the native form is shown on the left. Bound antibody was removed by washing with a high pH wash, as shown by the dip in response units following antibody binding. The tie2-EX-FLAG™ was then denatured using guanidium hydrochloride and 2-mercaptoethanol, and the ability of the antibodies to bind was again tested, as shown in the right column of Figure 9. The 1ell antibody no longer binds after denaturation of the tie2-EX-FLAG™, as shown by the absence of increase in response units; see top right-hand trace. The other two antibodies, 3g1 and 4g8, still show binding after denaturation of the tie2-EX-FLAG™, albeit at a low level, showing that although the conformation of the tie2-EX-FLAG™ is altered, it still remains attached to the chip.

The location of the FLAG™ peptide at the C-terminus did not adversely affect either experiments on the BIAcore™ or MAb production to the receptors. The purified NYK-EX-FLAG™ specifically bound the ligand VEGF. Also, there was no apparent binding from serum containing media, in contrast to our observation with alkaline phosphatase-fusion proteins. The apparent inertness of the FLAG™ peptide in protein interactions, compared with other markers such as alkaline phosphatase or the Fc portion of immunoglobulin, makes this system ideal for biosensor-based applications, since it avoids false positives.

### Example 2 Detection of VEGF in Serum

Mice were immunised with 1 x 10⁶ C6 glioma cells or 1 x 10⁶ U937 cells subcutaneously and the tumours allowed to develop to approximately 0.5-1.0 cm³. At this point blood samples were drawn from the animals and serum subsequently collected. The serum was assayed for binding to the NYK-FLAG derivatised chip and the amount of VEGF present estimated by comparison to a standard curve generated using recombinant human VEGF (Preprogen). Results are shown in Table 2 below.

**Table 2**

| Detection of VEGF in the Serum of Tumour Bearing Mice | | |
|---|---|---|
| | R.U. | VEGF (ng/ml) |
| Control #1 | 2.0 | <10 |
| Control #2 | 1.0 | <10 |
| Tumour #1 | 61.0 | 351 |
| Tumour #2 | 63.0 | 365 |
| rVEGF Run #1 | 1551 | 10,000 |
| | 598 | 3,125 |
| | 335 | 1,560 |
| | 154 | 780 |
| rVEGF Run #2 | 150 | 1,000 |
| | 83 | 500 |
| | 62 | 250 |
| | 29 | 125 |
| | 15 | 63 |

### Example 3 Detection of Recombinant VEGF

Recombinant VEGF (5 µl) was resuspended in 20 mM sodium acetate pH 4.5/0.02% Tween 20 and immobilised onto a standard BIAcore chip. Conditioned medium was flowed over the chip and bound material desorbed with 10 mM HC1. These results are shown below in Table 3.

**Table 3**

| Binding of NYK Extracellular Domain Construct to Recombinant VEGF Coupled to the Biosensor Chip. | |
|---|---|
| Sample | Relative Response (R.U.) |
| Medium | 53 |
| NYK extracellular domain | 1043 |
| anti-VEGF antibody | 2746 |

### Example 4 Detection of Monoclonal Antibodies 4H3 and 3B6 Binding to immobilised NYK-EX-FLAG

Monoclonal antibodies directed to the extracellular domain of VEGFR2 were used to detect the immobilised NYK-EX-FLAG on the BIAcore. Antibody (10 µl/ml) was applied to the chip and the relative response determined. The results are shown in Table 4 below.

**Table 4**

| Binding of Anti-VEGFR2 Monoclonal Antibodies to immobilised NYK-EX-FLAG | |
|---|---|
| Antibody | Relative Response (R.U.) |
| 4H3 | 1769 |
| 3B6 | 1194 |
| 4H3 (+3B6 already bound) | 1391 |

### Example 5 Bioassay for NYK Using An NYK-Erythropoietin Receptor Fusion Protein

Recent studies by Pacifici and Thomason (1994) suggest that most RTKs when transfected into factor-dependent cell lines such as FDCP-1 and BA/F3 do not produce a significant proliferative response to ligand. However, if a chimeric molecule comprising the extracellular domain of the RTK and the transmembrane and cytoplasmic domain of the cytokine receptor for erythropoietin receptor (EpoR) is used, the level of proliferation achieved is similar to that given by the wildtype cytokine receptor. This implies that aggregation of the receptor cytoplasmic domain is sufficient for some proliferative responses to be generated. The mechanism is illustrated in Figure 2.

Using site directed mutagenesis a silent mutation in the EpoR cytoplasmic domain was effected to remove a *Bgl*II restriction enzyme site at position 866 of the mouse Epo receptor published nucleotide sequence (D'Andrea *et al*, 1989). Another *Bgl*II site was then subsequently introduced at the junction of the extracellular domain and putative transmembrane domain of the EpoR. The cytoplasmic and transmembrane domain of the Epo receptor was then ligated in frame to the extracellular domain of the NYK receptor via the *Bgl*II site described in Examples 7 to 9. The insert was then subcloned into the pBOS expression vector via *Xba*I. This construct is designated pBOS-NYK/Epo.

pBOS-NYK/Epo was then cotransfected into the factor-dependent pre-B cell line BA/F3 with the neomycin resistance plasmid. The BA/F3 cell line is dependent on the presence of IL-3/GM-CSF for growth; removal of these factors results in rapid cell death within 24-48 h. Cells were selected in DMEM, 10% HI FCS, 50 µg/ml gentamicin, 20 µg/ml L-glutamine, 1.2 mg/ml G418 growth medium. Individual clones growing after 7-14 days were picked and expanded in liquid culture.

NYK/Epo expressing colonies were selected by two procedures:
(i) immunoprecipitation of ³⁵S-Met/Cys-labelled receptors with anti-NYK MAbs (prepared as described in our copending International Patent Application No. PCT/US95/01743 filed 9 February 1995) and analysis of SDS-PAGE;
(ii) stimulation of the cell line with VEGF.

1000 NYK/Epo-BA/F3 cells or non-expressing control BA/F3 cells were resuspended in a 15 µl volume of growth medium containing 10% medium conditioned by the growth of COS cells transfected with pCDM8-VEGF165. Stimulation of cells was judged over a period of 2-14 days when compared to control populations.

Three cell lines were demonstrated to respond to VEGF and to express the NYK-Epo chimera, as summarised in Table 5 and demonstrated by flow cytometry (Fig. 3). Flow cytometry analysis of VEGFR2-EpoR expression on the surface of BA/F3 cells was conducted as follows

BA/F3 cells (5 x 10⁵) transfected with the NYK-EpoR construct (BA/F3-NYK-EpoR#18) or cells transfected with vector alone (115) were reacted with monoclonal antibodies to the extracellular domain of the NYK receptor (4H3 described in PCT/US95/01727) or control antibodies directed to the tie2 receptor (PCT/US95/01743). After washing the cells were incubated with a goat anti-rat-FITC antibody, subsequently washed and the cells analysed by flow cytometry using a FACScan™ and CellQuest™ software (Becton Dickinson). The results are shown in Fig. 3. The traces are control cell line 115 stained with antibody 4H3 (----) and the BA/F3-NYK-EpoR cell line stained with either 4H3 ( 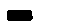 ) or control antibody 4g8 (_).

These cell lines were also shown to be specifically stimulated to grow by incubation with 10-20 µg/ml of MAbs directed to the extracellular domain of the NYK receptor. This is the result of cross-linking of receptors at the cell surface, which mimics ligand-induced dimerisation of the receptor.

The assay therefore specifically detects interactions of the NYK extracellular domain and its ligand VEGF, and is therefore useful for detection and evaluation of substances that may modulate this interaction, or detection and evaluation of inhibitors of such modulatory substances.

**Table 5**

| Stimulation of NYK-EpoR Transfected BA/F3 Cells | | | |
|---|---|---|---|
| Clone | WEHI3D conditioned medium | VEGF165 | Growth |
| BA/F3 | + | - | +++ |
| BA/F3 | - | + | - |
| BA/F3 vector only | - | + | - |
| BA/F3-NYK-EpoR#2 | - | + | ++ |
| BA/F3-NYK-EpoR#18 | - | + | ++ |
| BA/F3-NYK-EpoR#23 | - | + | ++ |

Transfected or untransfected BA/F3 cells were removed from WEHI3D conditioned medium, washed three to four times in PBS and resuspended in medium containing 5 ng/ml of VEGF165.

Growth was determined between 2 and 4 days in VEGF by visual inspection of cultures.

### Example 6 Detection of Mutant VEGF

Mutants were generated in the pCDNA-1 Amp expression vector. These were generated by using oligonucleotide directed mutagenesis by the technique already described. The mutations were either single or multiple amino acid substitutions in the wild type VEGF. The amino acid sequence of wild type VEGF used as a basis for these mutants is the same as the sequence for K73S shown in Figure 4 except that the wild type has a lysine (K) at position 73. The wild type leader sequence is also included in the mutants shown in Figure 4. Mutations were confirmed by sequencing. The sequence of the mutants is shown in Table 6.

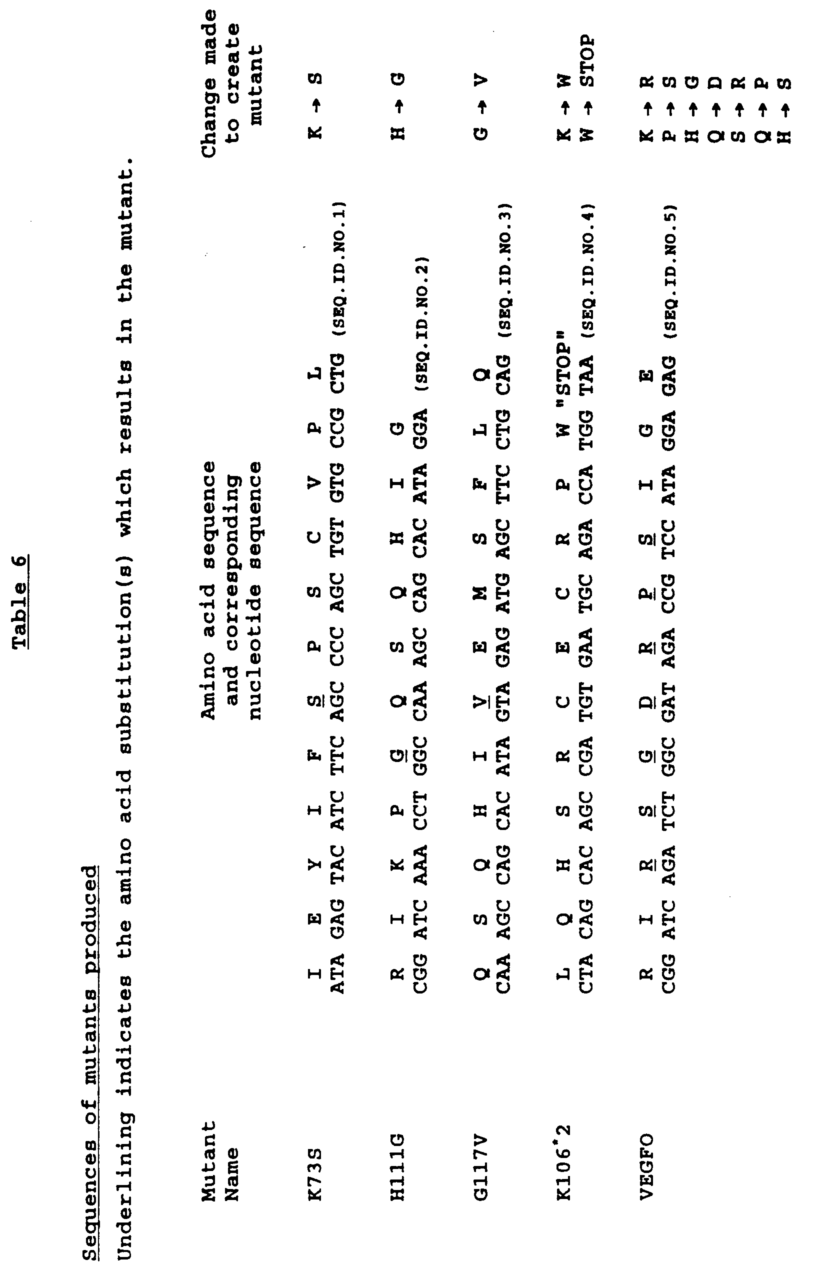

Constructs were transfected into COS cells by the DEAE-Dextran method and conditioned media collected for seven days post transfection. The conditioned media was diluted to 10% in the bioassay buffer as described previously in the presence of BA/F3-NYK-EpoR or Ba/F3 cells not expressing the receptor. After 48-72 hours the assay was assessed for the amount of proliferation and scored as 0 = no viable cells, 1 = less than 25% coverage of the well, 2 = 25-50% coverage of the well, 3 = 50-75% coverage of the well and 4 greater than 75% of the well covered. NT=not tested. The expression levels of the VEGF mutants was assessed by immunoprecipitation of 35S-Met/Cys labelled COS cell conditioned media with an antisera to VEGF and analysis by SDS-PAGE. The results are shown in Table 7.

**Table 7**

| Response of NYK-EpoR transfected BA/F3 cells to stimulation with conditioned medium from COS cells transfected with pCDM8 or PCDNA1-Amp containing VEGF wildtype or mutated forms generated by site-directed mutagenesis. | |
|---|---|
| **MUTANT** | **BIOASSAY** |
| Q62D | NT |
| D66S | 3 |
| E69S | NT |
| Y70H | 2 |
| E72T | 3 |
| K73S | 1 |
| N87G | 2 |
| E92H | 2 |
| H111G | 1 |
| I116Y | 2 |
| G117V | 1 |
| C2,4S | 2 |
| K106*2 | 4 |
| VEGF0 | 1 |
| PLGF WT | 0 |
| VEGF WT | 3 |
| VECTOR | 0 |

The above mutants were tested for their ability to induce or inhibit vascular permeability by the Miles Assay with the appropriate controls. This assay involves administering a blue dye to an animal which is distributed in the animal's blood vessels then intradermally injecting the animal with the mutant and observing the effect on vascular permeability.

The following examples relate to production of recombinant receptor for use in the above assays.

### Reagents

Purified M2 monoclonal antibody which recognises an internal epitope on the FLAG™ octapeptide (Cat# IB13025), M2-gel (IB13021) and free FLAG™ peptide (IB13070) were obtained from International Biotechnologies, New Haven, CT.

A cDNA clone encoding VEGF (165 amino acid form) was isolated by PCR from reverse transcribed mouse colon mRNA. The VEGF cDNA was sequenced and found to be identical to the known sequence (Breier *et al*, 1992). The fragment encoding VEGF was subcloned into the pEE6 vector and transfected into CHO cells as described below. Supernatants from stably-transfected CHO cells were collected and used for the NYK-EX-FLAG™ binding studies (see below). The bioactivity of VEGF produced in this manner was evaluated in a Miles vascular permeability assay (Miles and Miles, 1952) and the VEGF was found to be functional.

### Oligonucleotides

All oligonucleotides were synthesised by the Joint Protein Structure Laboratory of the Ludwig Institute for Cancer Research and The Walter and Eliza Hall Institute. The FLAG™ linker oligonucleotides were synthesised as two 39mers which were complementary over 35 nucleotides, giving a four base overhang corresponding to *Bgl*II cohesive ends (Figure 5). The oligonucleotides encoded the amino acid sequence of the FLAG™ octapeptide, an in-frame stop codon and a *Cla*I restriction enzyme site to allow selection of recombinants. Oligonucleotide linkers were phosphorylated with polynucleotide kinase using a conventional method, as previously described (Sambrook *et al*, 1989) and annealed at 37°C for 1 hour prior to ligation. Mutagenic oligonucleotides (27-35 mers) which introduced either *Bgl*II or *Bam*HI sites were made to sequences at the receptor extracellular/transmembrane border, as defined by hydrophobicity plots.

### Example 7 Site-directed Mutagenesis and Ligation of Linker Sequence

The strategy developed for isolating the extracellular domain of NYK, tie2 and RYK is summarised in Figure 6. Site-directed mutagenesis was used to generate mutant forms of the cDNA to enable the FLAG™ sequence, encoded in a set of oligonucleotides (Figure 5) to be placed in-frame, thereby creating the appropriate extracellular domain-FLAG™ fusion protein. Initially, site-directed mutagenesis was performed on single stranded DNA of the growth factor receptor to introduce either a *Bgl*II or *BamH*I site at the border of the extracellular and transmembrane regions using a mutagenic oligonucleotide. *BglII* enzyme sites were introduced into the NYK and RYK cDNAs, whereas a BamHI site was introduced into the *tie*2 cDNA due to an existing *BglII* site.

Full length cDNA clones encoding the mouse NYK/FLK-1/VEGFR2 receptor, mouse tie2 receptor and the human RYK receptor were subcloned into the mammalian expression vector pCDM8 (Invitrogen) using the *Bst*XI restriction enzyme site and *Bst*XI linkers. Single stranded UTP-containing DNA (Kunkel, 1985) was generated, and used as templates to make mutants of NYK, tie2 and RYK cDNA which encoded the required restriction enzyme sites. Mutant receptor cDNAs containing *Bgl*II or *BamH*I sites at their extracellular domain and the transmembrane domain boundaries (as determined by hydrophobicity plots (Kyte and Dolittle, 1982) using the DNA STAR™ software) were digested with the appropriate restriction enzyme, phosphatased and ligated with the oligonucleotide linker sequence encoding the FLAG™ marker peptide (IBI);
^{5'}GATCTGACTACAAGGACGACGACGATGACAAGTGAATCGATA^{3'},
(N)Asp-Tyr-Lys-Asp-Asp-Asp-Asp-Lys-Term(C)).
A unique *Cla*I site was installed at the 3' end of the oligonucleotides, and recombinants were selected by *Cla*I restriction enzyme digestion. All clones were sequenced to ensure that no other mutations had been introduced, and that the FLAG™ sequence was in the correct orientation. The FLAG™ fusion proteins were designated NYK-EX-FLAG, tie2-EX-FLAG™ and RYK-EX-FLAG™, respectively.

The small phosphorylated FLAG™ linker oligonucleotides (39 mers) ligated easily into the digested vector fragments, and recombinants were accurately detected by use of the internal *Cla*I site.

### Example 8 Cell Culture

COS cells were maintained in RPMI-1640 medium with 10% FCS and 50 µg/ml gentamicin at 37°C and 5% CO₂. COS cells were transfected by the DEAE dextran method as described previously (Aruffo and Seed, 1987).
pEE6-NYK-EX-FLAG™, pEE6-tie2-EX-FLAG™ and pEE6-RYK-EX-FLAG™ constructs were transfected into CHO-K1 cells by calcium phosphate precipitation and selected in Glasgow Modified Eagle's Medium (without L-glutamine, without NaHCO₃, Cytosystems, Castle Hill, N.S.W.) supplemented with 10% fetal calf serum, 50 µg/ml gentamicin, non-essential amino acids, sodium pyruvate and 25 mM methionine sulphoxide (MSX). Bulk production of the protein was achieved by culturing the cells on Cytodex-3 beads (Pharmacia, Uppsala, Sweden) in roller bottles in the presence of 2 mM sodium butyrate. Cells were grown until they reached about 80-90% confluence. Conditioned medium was collected every week by replacing with fresh medium. Supernatants were harvested from the roller bottles 16-20 days after seeding and the following cocktail of protease inhibitors was added:
0.02% NaN₃, 1 mM PMSF, 0.2 TIU/ml aprotinin,
10 µg/ml pepstatin and 0.5 mM EDTA. The supernatants were either used directly or stored at -70°C prior to use.

The constructs and controls were transfected into COS cells and supernatants were analysed for fusion protein three days post-transfection. Biosynthetic labelling of the cells, immunoprecipitation with M2-gel and SDS-PAGE analysis demonstrated products of the expected size, as shown in Figure 7. Diffuse bands in the range of 120-130 kD for NYK-EX-FLAG™ (Figure 7A) and 100-110 kD for tie2-EX-FLAG™ (Figure 7B) were specifically immunoprecipitated with the M2-gel. Predicted sizes for the NYK-EX-FLAG™, tie2-EX-FLAG™ and RYK-EX-FLAG™ fusion proteins including the contribution of N-linked glycosylation, as predicted from immunoprecipitation of the mature cell surface receptors (Runting *et al*, 1993; Stacker *et al*, 1993), are 125,000 Daltons, 95,000 Daltons and 38,000 Daltons respectively.

These initial studies in COS cells have shown that (i) the FLAG™ peptide can be expressed at the C-terminus of the receptor extracellular domains, (ii) the FLAG™-fusion protein is secreted by the cell, and (iii) the FLAG™-fusion protein can be recognised effectively by the M2 antibody.

The constructs were than subcloned into the CHO cell expression vector pEE6 and transfected into CHO cells. Supernatants of stably transfected CHO cells were screened for expression of fusion proteins by immunoprecipitation following biosynthetic labelling. Clones that produced fusion proteins of the predicted size were selected, and large scale production of the secreted protein undertaken. Expression levels of transfected CHO cells in 25 mM MSX were sufficient (0.5-1.0 µg/ml), so that selection in higher amounts of MSX was not attempted. CHO cells were grown on Cytodex-3 beads in roller bottles and produced in the range of 1 µg of fusion protein per ml of expended tissue culture supernatant.

### Example 9 Purification of Fusion Proteins

NYK-EX-FLAG™, tie2-EX-FLAG™ and RYK-EX-FLAG™ were purified from medium conditioned by transfected CHO cells using affinity chromatography on M2 (anti-FLAG™) gel (IBI). A separate column was used for each receptor construct. Supernatant (100-200 ml) was passed over a 1-2 ml column of M2-gel which was subsequently washed with 10 volumes of 10 mM Tris-HCl pH 8.0, 150 mM NaCl, 0.02% Tween 20; 10 volumes of 50 mM triethylamine (TEA) pH 10.0, 150 mM NaCl, 0.02% Tween 20 and again with 10 volumes of 10 mM Tris-HCl pH 8.0, 150 mM NaCl, 0.02% Tween 20. This step may not be necessary, depending on the yield and purity of protein which is required. Bound material was eluted with either 100 mM glycine-HCl pH 3.0, 0.02% Tween (neutralised in 1/10 volume 1 M Tris-HCl pH 8.6) or 25-50 µg/ml FLAG™ peptide (N-Asp-Tyr-Lys-Asp-Asp-Asp-Asp-Lys-C) in 10 mM Tris-HCl pH 8.0, 150 mM NaCl, 0.02% Tween 20. Columns were eluted in 1 ml fractions, and 10 µl samples combined with 10 µl of reducing SDS-PAGE sample buffer were analysed by SDS-PAGE and proteins detected by silver staining. Following desorption with free peptide the affinity resin was treated with 0.1 M glycine pH 3.0 to remove bound peptide prior to the next purification cycle. For coupling to the sensor chip and depletion of free FLAG™ peptide, fusion proteins were further purified by high-performance liquid chromatography in a Tris-free buffer system (Nice *et al*, 1994) on a µMonoQ PC 1.615 anion exchange column (Pharmacia) to give a single homogeneous species corresponding to either NYK-EX-FLAG™, tie2-EX-FLAG™ or RYK-EX-FLAG™.

The strategy developed for purification of the fusion proteins from medium conditioned with transfected CHO cells involved a single immunoaffinity chromatography step on an M2-antibody column (2 ml of packed beads per 100 ml of CHO cell supernatant). The proteins were eluted from the column with excess free FLAG™ peptide, and further purification to homogeneity was achieved by microbore anion exchange HPLC. This also enabled the removal of excess free FLAG™ peptide. Desorption of non-specific proteins was achieved by a two-step washing procedure involving buffers of pH 8.0 and pH 10.0 respectively. This protocol appeared sufficient to remove the large majority of contaminants, as judged by SDS-PAGE analysis of the purified fractions, which is illustrated in Figure 8. Purification of NYK-EX-FLAG™ and tie2-EX-FLAG™ gave predominant bands in the size range of 120-130 kD and 100-110 kD respectively (Figure 8A and 8B). Purification and analysis of RYK-EX-FLAG™ gave in addition to the predicted 35-40 kD species a range of other higher molecular weight proteins (Figure 8C). These are consistently present in all RYK-EX-FLAG™ purifications, and may represent oligomerised forms of the fusion protein. Competition with 25-50 µg/ml of FLAG™ peptide was sufficient to remove the bound extracellular domain-FLAG™, as further elution of the column with 0.1 M glycine-HCl pH 3.0 gave little if any fusion protein (Figure 8A-C). Interestingly, elution with low pH yielded more contaminating proteins in the samples than elution with FLAG™ peptide alone. Figure 8B (lanes 6-9) shows a range of contaminants in the low-pH eluate that were not seen with the peptide eluted material. We conclude that the FLAG™ peptide is relatively specific in its ability to elute only FLAG™-M2 interactions, and does not dissociate most other non-specific interactions. We detected a small amount of high molecular weight material, presumably immunoglobulin, eluting from the column after pH 3.0 treatment; this was not eluted with the peptide at pH 8.0. Fractionation of the M2-affinity eluate on a µmonoQ column effectively removed the free FLAG™ peptide, which could be recycled for future use. Our overall yield of HPLC purified extracellular domain from the CHO cell conditioned medium was calculated to be in the order of 80 µg of protein per 100 ml.

To confirm that the material eluting from the M2 affinity column was in a native conformation, the NYK-EX-FLAG™ fusion protein was tested for its ability to bind a known ligand, vascular endothelial growth factor (VEGF) (Millauer *et al*, 1993) as described in Example 1.

### Advantages of the Invention

In this study we have described a protocol for isolating functional extracellular domains of receptor tyrosine kinases using a combination of site-directed mutagenesis, the FLAG™ peptide system and CHO cell expression. Our need to develop this method was initially prompted by a complete lack of reagents for characterising the proteins encoded by three recently isolated cDNA clones. The advantage of our protocol is that it allows for the precise positioning of the FLAG™ sequence at the junction of the extracellular and predicted transmembrane domain of a nominated receptor. *Bgl*II/*BamH*I restriction enzyme sites were used to introduce the FLAG™ sequence because of their compatibility with each other and the absence of at least one of these in the cDNA clones being studied. In addition, the strategy provides a universal adaptor site whereby the receptor extracellular domain can be ligated to other proteins of interest. For example, the extracellular domain could be ligated into the vector AP-tag-1 (Flanagan and Leder, 1990) for expression of fusion proteins with secreted alkaline phosphatase or any other protein domain to which an in-frame, compatible restriction enzyme site has been incorporated. For example, a fusion protein in which the extracellular domain of NYK protein is linked to the cytoplasmic and transmembrane domain of the erythropoietin receptor is described in a concurrently-filed application.

The pCDM8 vector was used as the base vector for our technique, as it can be used for generating single stranded DNA for site-directed mutagenesis, as well as for transient expression in COS cells. This allows the rapid testing of constructs for expression of the fusion protein before proceeding to large scale production. Subcloning of the final constructs into the CHO cell expression vector pEE6 is also simple, due to compatible *Xba*I sites present in the pCDM8 and pEE6 polylinkers. We have used site-directed mutagenesis to introduce the necessary restriction enzyme sites at the junction of the extracellular and transmembrane domains. This technique has some advantages over PCR, especially when attempting to generate large fragments, or when oligonucleotide primers are derived from sequences possessing GC-rich regions, as frequently occurs in the 5' regions of cDNAs encoding RTKs (Kozak, 1991). Alternatively, PCR could be used to generate smaller fragments encoding the *Bgl*II/*BamH*I sites and then ligated to the extracellular domain cDNA via an appropriate restriction enzyme site.

A major advantage of this system is the commercial availability of the FLAG™ system components, and their effectiveness in yielding relatively pure fusion protein preparations with a one-step procedure from starting material containing 10% fetal calf serum. The ability to elute with the free FLAG™ peptide is also a major advantage, maximising the likelihood of obtaining native receptor extracellular domain by avoiding the need for exposure to extreme pH or to chaotropic agents. We could not make use of the divalent cation-dependent nature of the M1 MAb for mild elution, as this MAb requires a free FLAG™ N-terminus for recognition; therefore the M2 antibody was utilised. Mild elution is important when dealing with receptors which possess uncharacterised ligands, as the integrity of the ligand binding site cannot be readily assessed.

The location of the FLAG™ peptide at the C-terminus did not adversely affect either experiments on the BIAcore™ or MAb production to the receptors. The purified NYK-EX-FLAG™ specifically bound the ligand VEGF. Also, there was no apparent binding from serum containing media, in contrast to our observation with alkaline phosphatase-fusion proteins. The apparent inertness of the FLAG™ peptide in protein interactions, compared with other markers such as alkaline phosphatase or the Fc portion of immunoglobulin, makes this system ideal for biosensor-based applications, since it avoids false positives.

Our method permits the isolation of purified, functional receptor extracellular domain-FLAG™ fusion proteins using a mammalian expression system. Utilising such a system means that glycosylation and protein folding are most likely to resemble the native state. Linkage of a well-characterised marker peptide means that reagents are available to affinity purify reasonable quantities of the fusion protein, which will facilitate further study. The size and C-terminal location of the FLAG™ peptide means that the effects on the protein being studied are minimised. Other workers have successfully expressed a FLAG™ (N-terminus)-human platelet-activating receptor fusion protein in mammalian cells (COS-7) to demonstrate its topology at the membrane, although studies examining the function of the receptor were not performed (Gerard and Gerard, 1990). This is the first report of the FLAG™ peptide as a C-terminal marker in a mammalian expression system. FLAG™ was designed originally to be linked to the N-terminal of the desired protein, and has a structure such as to permit ready cleavage from this N-terminal (U.S. Patent No. 4,703,004).

This type of approach is generally applicable to the examination of cell surface receptors, in particular for workers intending to isolate the cognate ligands of these receptors. Although the FLAG™ system itself is not inexpensive, it compares favourably with the development costs of a specific MAb affinity column, for which one would still have the problem of elution conditions to contend with. The time scale of the present technique is about two to four months from isolation of a clone encoding the extracellular domain to having microgram-milligram quantities of purified fusion protein.

It will be apparent to the person skilled in the art that while the invention has been described in some detail for the purposes of clarity and understanding, various modifications and alterations to the embodiments and methods described herein may be made without departing from the scope of the inventive concept disclosed in this specification.

References cited herein are listed on the following pages, and are incorporated herein by this reference.

### REFERENCES

Aruffo A. and B. Seed.
   "Molecular cloning of a CD28 cDNA by a high-efficiency COS cell expression system"
   Proc. Natl. Acad. Sci. U.S.A., 1987 84 8573-8577
Breier G., U. Albrecht, S. Sterrer and W. Risau.
   "Expression of vascular endothelial growth factor during embryonic angiogenesis and endothelial cell differentiation"
   Development, 1992 114 521-532
Fanslow W.C., D. Anderson, K.H. Grabstein, E.A. Clark, D. Cosman and R.J. Armitage.
   "Soluble forms of CD40 inhibit biological responses of human B cells"
   J. Immunol., 1992 149 655-660.
Flanagan J.G. and P. Leder.
   "The kit ligand: a cell surface molecule altered in steel mutant fibroblasts"
   Cell, 1990 63 185-194.
Gerard N.P. and C. Gerard.
   "Construction and expression of a novel recombinant anaphylatoxin, C5a-N19, as a probe for the human C5a receptor"
   Biochemistry, 1990 29 9274-9281.
D'Andrea, A.D., H.F. Lodisch and G.G. Wong
   "Expression cloning of the murine erythropoietin receptor"
   Cell, 1989 57 277-285
Hopp T.P., K.S. Prickett, V.L. Price, R.T. Libby, C.J. March, D.P. Cerretti, D.L. Urdal and P.J. Conlon.
   "A short polypeptide marker sequence useful for recombinant protein identification and purification.
   Biotechnology, 1992 6 1204-1210.
Hovens C.M., S.A. Stacker, A. Andres, A.G. Harpur, A. Ziemiecki and A.F. Wilks.
   "RYK, a receptor tyrosine kinase-related molecule with unusual kinase domain motifs"
   Proc. Natl. Acad. Sci. U.S.A., 1992 89 11818-11822.
Kozak M.
   "An Analysis of Vertebrate mRNA Sequences: Intimations of Translational Control"
   J. Cell Biol., 1991 115 887-903.
Kunkel T.A.
   "Rapid and efficient site-specific mutagenesis without phenotypic selection"
   Proc. Natl. Acad. Sci. U.S.A., 1985 82 488-492.
Kyte J. and R.F. Doolittle.
   "A simple method for displaying the hydropathic character of a protein"
   Journal of Molecular Biology, 1982 157 105-132.
Li M., Y.N. Jan and L.Y. Jan.
   "Specification of subunit assembly by the hydrophobic amino-terminal domain of the shaker potassium channel"
   Science, 1992 257 1225-1230.
Miles A.A. and E.M. Miles.
   "Vascular reactions to histamine, histamine-liberator and leukotaxine in the skin of guinea-pigs"
   Journal of Physiology (London), 1952 118 228-257.
Millauer B., S. Wizigmann-Voos, H. Schnurch, R. Martinez, N.P.H. Moller, W. Risau and A. Ullrich.
   "High affinity VEGF binding and developmental expression suggest Flk-1 as a major regulator of vasculogenesis and angiogenesis"
   Cell, 1993 72 835-846.
Nice E., M. Lackmann, F. Smyth, L. Fabri and A.W. Burgess.
   "Synergies between micropreparative high-performance liquid chromatography and an instrumental optical biosensor"
   Journal of Chromatography, 1994 660 169-185.
Oelrichs R.B., H.H. Reid, O. Bernard, A. Ziemiecki and A.F. Wilks.
   "*NYK/FLK-1*: A putative receptor protein tyrosine kinase isolated from E10 embryonic neuroepitheium is expressed in endothelial cells of the developing embryo"
   Oncogene, 1993 8 11-18.
Pacifici R.E. and A.R. Thomason
   "Hybrid tyrosine kinase/cytokine receptors transmit mitogenic signals in response to ligand"
   J. Biol. Chem., 1994 269 1571-1574.
Paul S.R., D. Merberg, H. Finnerty, G.E. Morris, J.C. Morris, S.S. Jones, R. Kriz, K.J. Turner and C.R. Wood.
   "Molecular cloning of the cDNA encoding a receptor tyrosine kinase-related molecule with a catalytic region homologous to c-met. International Journal of Cell"
   Cloning, 1992 10 309-314.
Runting A.S., S.A. Stacker and A.F. Wilks.
   "*tie2*, a putative protein tyrosine kinase from a new class of cell surface receptor"
   Growth Factors, 1993 9 99-105.
Sambrook J., E.F. Fritsch and T. Maniatis.
   "Molecular Cloning A Laboratory Manual"
   Cold Spring Harbor Laboratory Press, 1989.
Smith D.B. and K.S. Johnson.
   "Single-step purification of polypeptides expressed in *E.coli* as fusions with glutathione S-transferase"
   Gene, 1988 67 31-40.
Stacker S.A., C.M. Hovens, A. Vitali, M.A. Pritchard, E. Baker, G.R. Sutherland and A.F. Wilks.
   "Molecular cloning and chromosomal localisation of the human homologue of a receptor related to tyrosine kinases (ryk)"
   Oncogene, 1993 8 1347-1356.
Su X., A.K. Prestwood and R.A. McGraw.
   "Production of recombinant porcine tumour necrosis factor alpha in a novel *E.coli* expression system"
   Biotechniques, 1992 13 756-762.
Terman B.I., M. Dougher Vermazen, M.E. Carrion, D. Dimitrov, D.C. Armellino, D. Gospodarowicz and P. Bohlen.
   "Identification of the KDR tyrosine kinase as a receptor for vascular endothelial cell growth factor"
   Biochem. Biophys. Res. Commun., 1992 187 1579-1586.
Ullrich A. and J. Schlessinger.
   "Signal transduction by receptors with tyrosine kinase activity"
   Cell, 1990 61 203-212.
Wilks A.F.
   "Two putative protein-tyrosine kinases identified by application of the polymerase chain reaction"
   Proc. Natl. Acad. Sci. U.S.A., 1989 86 1603-1607.
Zhang X., K.N. Wills, M. Husmann, T. Hermann and M. Pfahl.
   "Novel pathway for thyroid hormone receptor action through interaction with *jun* and *fos* oncogene activities"
   Mol. Cell Biol., 1991 11 6016-6025.

## Claims

1. Ligand capable of binding the receptor protein NYK detected by the method comprising contacting a NYK receptor protein, a derivative thereof or a functional equivalent thereof with a putative ligand under conditions suitable to allow binding of said putative ligand to said receptor, derivative or equivalent thereof and determining whether binding has occurred.

2. A pharmaceutical composition comprising the preparation of Claim 1 and a pharmaceutically acceptable carrier or diluent.

3. Molecule which is capable of inhibiting interaction of the receptor protein NYK and a ligand therefor detected by the method comprising contacting a molecule which is suspected of inhibiting said interaction with
(i) NYK, a derivative thereof or a functional equivalent thereof, and
(ii) a known ligand of NYK
under conditions suitable to allow binding of said NYK, derivative thereof or functional equivalent thereof to said known ligand and determining whether binding has occurred.

4. A pharmaceutical composition comprising the preparation of Claim 3 and a pharmaceutically acceptable carrier or diluent.

5. Molecule which has an increased ability to stimulate activity of the receptor protein NYK as compared to a native ligand of NYK detected by the method comprising
(i) contacting a molecule which is suspected of having said increased ability with NYK, a derivative thereof or a functional equivalent thereof under conditions suitable to allow binding of said NYK, derivative thereof or functional equivalent thereof with said molecule;
(ii) determining whether binding has occurred; and
(iii) comparing said determination with the amount of binding in a control wherein said control comprises contacting (i) and a native ligand of NYK under suitable conditions.

6. A pharmaceutical composition comprising the preparation of Claim 5 and a pharmaceutically acceptable carrier or diluent.

7. An isolated polypeptide which is a mutant of wild type VEGF and is capable of inhibiting or promoting the activity of the receptor protein NYK.

8. The polypeptide of Claim 7 wherein said polypeptide comprises amino acid deletion or substitution in the equivalent of the cysteine-knot motif of wild type VEGF.

9. The polypeptide of Claim 7 wherein said polypeptide comprises one or more amino acid substitutions in the region which corresponds to the V3 domain of wild type VEGF.

10. The polypeptide of Claim 7 wherein said polypeptide comprises a mutation at least one position corresponding to mouse VEGF, said position selected from the group consisting of positions 73, 111, 117, 109, 110, 111, 112, 113, 114, and 115.

11. The polypeptide of Claim 7 wherein said polypeptide is selected from the group consisting of K73S, 811G, G117V, VEGF0 and variants thereof having substantially the same biological activity.

12. The polypeptide of Claim 7 comprising an agonist of the receptor protein NYK and a truncated form of wild type VEGF with one or more amino acid substitutions in the region which corresponds to the V3 region of wild type VEGF.

13. The polypeptide of Claim 12 wherein said tuncation is at or about residue 133 corresponding to wild type mouse VEGF, and wherein said polypeptide has an amino acid substitution at or around the same position.

14. A pharmaceutical composition comprising the polypeptide of Claim 7 and a pharmaceutically acceptable carrier or diluent.

15. An isolated nucleic acid molecule which encodes a polypeptide capable of inhibiting or promoting the activity of the receptor protein NYK.

16. The molecule of Claim 15 wherein said molecule has a nucleotide sequence which encodes a polypeptide with amino acid deletions or insertions in the equivalent of the cysteine-knot motif of wild type VEGF.

17. The molecule of Claim 15 wherein said molecule has a nucleotide sequence which encodes a polypeptide with one or more substitutions in the region which corresponds to the V3 domain of a wild type VEGF.

18. The molecule of Claim 17 wherein said molecule encodes a polypeptide with a mutation at least one position, said position selected from the group consisting of positions 111, 117, 109, 110, 111, 112, 113, 114, and 115 corresponding to wild type mouse 3VEGF.

19. The molecule of Claim 18 wherein said molecule encodes a polypeptide selected from the group consisting of K73S, H111G, G117V, VEGF0 and variants thereof having substantially and same biological activity.

20. The molecule of Claim 15 wherein said molecule encodes a truncated protein terminating at or around residue 133 corresponding to wild type mouse VEGF and having an amino acid substitution at around the same position.

21. A method of producing a recombinant protein having the biological activity of an extracellular domain of a receptor protein tyrosinase kinase selected from the group consisting of *tie*2 and RYK, comprising the steps:
(a) generating a restriction site in the nucleic acid encoding the protein at the junction of the extracellular and predicted transmembrance domains of the receptor, wherein the restriction site is chosen such that it will be unique to a construct;
(b) ligating to the nucleic acid produced in step a) an oligonucelotide sequence encoding a marker peptide and an inframe stop codon;
(c) expressing the construct produced in step b) in a mammalian host cell in culture, to obtain a protein; and
(d) isolating said protein by using an affinity reagent specific for the marker peptide.

22. The method of Claim 21 wherein said protein is purified.

23. The method of Claim 21 wherein step a) is carried out using site-directed mutagenesis or using polymerase chain reaction.

24. The method of Claim 21 wherein the restriction site is *Bgl*II or *Bam*HI.

25. The method of Claim 21 wherein the unique restriction site introduced in step b) is *Cla*1.

26. The method of Claim 21 wherein the marker peptide is FLAG™.

27. The method of Claim 21 wherein the marker peptide is inserted at the C-terminal end of the extracellular domain.

28. The method of Claim 22 wherein the purification is carried out using anti-FLAG™ peptide affinity gel and mild elution with free FLAG™ peptide.

29. The method of Claim 21 wherein the host cells are CHO cells.

30. An extracellular RTK domain produced by the method of Claim 21.

31. A conmposition comprising the extracellular RTK domain of Claim 30, and a pharmaceutically acceptable carrier or diluent.

32. An isolated nucleic acid molecule generated by the method of Claim 21.

33. A monoclonal antibody directed against an extracellular RTK domain of Claim 30.
